# EUROPÄISCHE PATENTSCHRIFT

(11) **EP 2 009 014 B1**
(45) Veröffentlichungstag und Bekanntmachung des Hinweises auf die Patenterteilung: **24.10.2018**
(21) Anmeldenummer: 07012228.8
(22) Anmeldetag: 22.06.2007
(51) Int. Cl.: C07D 487/16, C09K 11/06, H01L 51/50, H01L 51/00, H05B 33/14

(54) **Verwendung eines Precursors eines n-Dotanden zur Dotierung eines organischen halbleitenden Materials, Precursor und elektronisches oder optoelektronisches Bauelement**
Application of a precursor of an n-dopant for doping an organic semi-conducting material, precursor and electronic or optoelectronic component
Utilisation d'un précurseur d'un n-dopant destiné au dopage d'un matériau semi-conducteur organique, précurseur et composant électronique ou optoélectronique

(43) Veröffentlichungstag der Anmeldung: 31.12.2008
(73) Patentinhaber: Novaled GmbH, 01307 Dresden (DE)
(72) Erfinder: Limmert, Michael, 01307 Dresden (DE); Lux, Andrea, 01307 Dresden (DE); Hartmann, Horst, 01307 Dresden (DE)
(74) Vertreter: Bittner, Thomas L.

(56) Entgegenhaltungen:
- EP-A- 1 508 903
- WO-A-03/070822
- WERNER A G ET AL: "Pyronin B as a donor for n-type doping of organic thin films" APPLIED PHYSICS LETTERS, AIP, AMERICAN INSTITUTE OF PHYSICS, MELVILLE, NY, US, Bd. 82, Nr. 25, 23. Juni 2003 (2003-06-23), Seiten 4495-4497, XP012034443 ISSN: 0003-6951
- VAID T P ET AL: "Investigations of the 9,10-diphenylacridyl radical as an isostructural dopant for the molecular semiconductor 9,10-diphenylanthracene" CHEMISTRY OF MATERIALS, AMERICAN CHEMICAL SOCIETY, WASHINGTON, US, Bd. 15, Nr. 22, 7. Oktober 2003 (2003-10-07), Seiten 4292-4299, XP002355671 ISSN: 0897-4756
- YOKOI H ET AL: "Photochemical Doping of TCNQ by Photoinduced Electron Transfer and C-C Cleavage of Radical Cation" CHEMISTRY LETTERS, NIPPON KAGAKUKAI, TOKYO, JP, Bd. 3, 1. Mai 2003 (2003-05-01), Seiten 241-242, XP002355670 ISSN: 0366-7022

## Beschreibung

Die vorliegende Erfindung betrifft eine Verwendung eines Precursors eines n-Dotanden zur Dotierung eines organischen halbleitenden Materials, als Blockerschicht, als Ladungsinjektionsschicht, als Elektrodenmaterial, als Speichermaterial oder als Halbleiterschicht selbst in elektronischen oder optoelektronischen Bauelementen, entsprechende Precursor, organisches halbleitendes Material sowie elektronische oder optoelektronische Bauelemente.

Es ist bekannt, organische Halbleiter durch Dotierung hinsichtlich ihrer elektrischen Eigenschaften, insbesondere ihrer elektrischen Leitfähigkeit, zu verändern, wie dies auch bei anorganischen Halbleitern, wie Siliciumhalbleitern, der Fall ist. Hierbei wird durch Erzeugung von Ladungsträgern im Matrixmaterial eine Erhöhung der zunächst recht niedrigen Leitfähigkeit sowie je nach Art des verwendeten Dotanden eine Veränderung im Fermi-Niveau des Halbleiters erreicht. Eine Dotierung führt hierbei zu einer Erhöhung der Leitfähigkeit von Ladungstransportschichten, wodurch ohmsche Verluste verringert werden, und zu einem verbesserten Übergang der Ladungsträger zwischen Kontakten und organischer Schicht. Die Dotierung im Leitfähigkeitssinne ist durch einen Ladungsübertrag vom Dotand auf ein nahe liegendes Matrixmolekül gekennzeichnet (n-Dotierung, Elektronenleitfähigkeit erhöht), bzw. durch den Übertrag eines Elektrons von einem Matrixmolekül auf einen nahe liegenden Dotanden (p-Dotierung, Löcherleitfähigkeit erhöht). Der Ladungsübertrag kann unvollständig oder vollständig erfolgen und lässt sich z.B. durch die Interpretation von Schwingungsbanden einer FTIR-Messung (fourier-transformed infrared-spectroscopy) bestimmen.

Die bisher verwendeten anorganischen Dotanden, wie Alkali- oder Erdalkalimetalle (z.B. Cäsium) oder Lewis-Säuren (z.B. FeCl₃), sind bei organischen Matrixmaterialien aufgrund ihrer hohen Diffusionskoeffizienten meist nachteilig, da die Funktion und Stabilität der elektronischen Bauelemente beeinträchtigt wird. Mit diesen anorganischen Dotanden sind weiterhin Schwierigkeiten in der Produktion verbunden, da sie in der Regel einen hohen Dampfdruck bei Raumtemperatur aufweisen und in Vakuumprozessen die Produktionsanlagen kontaminieren können. Insbesondere Alkali- und Erdalkalimetalle haben den weiteren Nachteil, dass ihre Verwendung durch ihre starke Reaktivität mit Luft erschwert ist. Diese bekannten Nachteile sollen mit Hilfe molekularer n-Dotanden vermieden werden.

Starke molekulare Dotanden können direkt wirkende Dotanden sein (also solche, die wie Alkalimetalle ihre Wirkung unmittelbar und ohne weitere aktivierende Maßnahme entfalten; siehe oben). Beispiele hierfür sind Tetrathiafulvalene oder auch Decamethylcobaltocen (WO 03/088271 A). Diese Substanzen weisen jedoch als bedeutsamen Nachteil eine ausgeprägte Empfindlichkeit gegenüber Atmosphärilien auf (Sauerstoff und Wasser), so dass bei ihrer Anwendung in der Regel besondere Maßnahmen getroffen werden müssen (z.B. Inertgasatmosphäre).

Eine Alternative dazu besteht in der Verwendung einer geeigneten Vorläuferverbindung (im Folgenden "Precursor" genannt), die bei geeigneter Aktivierung, beispielsweise durch Anregung mit Licht, die aktive Spezies freisetzt. Beispiele hierfür sind Dimere des Diphenylmethylradikals (H. Yokoi et al., Chem. Lett. 1999, 241-242) oder auch Leukokristallviolett (DE 10307125 A1). Hierbei ist in der Regel die effektive Dotierwirkung jedoch relativ gering; insbesondere können mit Diphenylmethylradikalen nur ausgewiesene Akzeptoren dotiert werden. Eine besonders wirkungsvolle Art von Dimeren sind Bipyridinyle und insbesondere Biimidazolyle (J. Ludvik, F. Pragst et al., J. Elelctroanal. Chem. Interfac. Electrochem.1984, 180(1-2), 141-156). Hierbei besteht jedoch die Schwierigkeit des synthetischen Zugangs, der bei diesen Materialien elektrochemische Expertise verlangt. Diese besondere Technik stellt wiederum einen bedeutenden limitierenden Faktor für die technische Produktion dar, weil Elektrolysen, von prominenten Ausnahmen wie der Schmelzflusselektrolyse abgesehen, bei Feinchemikalien kaum Anwendung finden.

Dass Imidazoline, genauer 2H-Imidazoline, prinzipiell dotierfähig sein können, wird in E. Hasegawa et al. J. Org. Chem. 2005, 70, 9632-9635 beschrieben. Hier wird ein Elektronentransfer nach optischer Anregung als erster Schritt einer komplexen Reaktionssequenz postuliert. D.h. aber auch, dass für einen n-Dotanden eine Struktur gefunden werden muss, die diese Folgereaktionen möglichst unterbindet und damit den Dotiereffekt dauerhaft erhält.

EP 1 508 903 A1 beschreibt dotierte organische halbleitende Materialien und Verfahren zur Herstellung derselben.

WO 03/070822 A beschreibt dotierte organische halbleitende Materialien und ein Verfahren zur Herstellung derselben.

Werner et al., Applied Physics Letters, 2003, 82(25), 4495, beschreibt die Verwendung von Pyronin B als Donor zum n-Dotieren von organischen dünnen Schichten.

Vaid et al., Chemistry of Materials, 2003, 22(7), 4292 beschreibt 9,10-Diphenylacridyl Radikale als isostruktureller Dotand für den molekularen Halbleiter 9,10-Diphenylanthracen.

Yokoi et al., 2003, 3, 241 beschreibt das photochemische Dotieren von TCNQ durch photoinduzierten Elektronentransfer und C-C-Spaltung von Radikalkationen.

Der vorliegenden Erfindung liegt die Aufgabe zugrunde, die Nachteile aus dem Stand der Technik zu überwinden und eine Möglichkeit bereitzustellen, auf einfache und effektive Weise eine n-Dotierung eines organischen halbleitenden Materials bereitzustellen, wobei der erhaltene n-Dotand eine hohe Donizität (effektive Dotierstärke) aufweist und darüber hinaus leicht modifizierbar ist. Ferner sollen neue Verbindungen bereitgestellt werden, die als ein entsprechender Precursor eines n-Dotanden geeignet sind. Ferner sollen organische, halbleitende Materialien sowie elektronische und optoelektronische Bauelemente bereitgestellt werden.

Diese Aufgabe wird gelöst durch den Gegenstand der unabhängigen Ansprüche. Bevorzugte Ausführungsformen ergeben sich aus den Unteransprüchen.

Überraschenderweise wurde festgestellt, daß die Nachteile aus dem Stand der Technik überwunden werden können, wenn die Precursor eines n-Dotanden zur Dotierung eines organischen halbleitenden Materials und für die anderen Zwecke eingesetzt werden, wie sie in Anspruch 1 aufgeführt sind. Bei diesen Precursern wird durch geeignete (zumeist optische) Anregung eine labile Bindung irreversibel gespalten. Bei den erfindungsgemäß eingesetzten Verbindungen wird bei Anregung der Substituent L als neutrales Radikal abgegeben (Abgangsgruppe). Dadurch wird der eigentliche Dotand, das intermediär entstandene Radikal, freigesetzt, welcher aufgrund seiner 7 π-Elektronenstruktur ein sehr effektives Reduktionsmittel darstellt: es ist bestrebt, sein überschüssiges Elektron unter Bildung eines sehr stabilen, aromatischen Kations abzugeben. Rezipient des Elektrons ist bevorzugt ein schwacher Akzeptor (beispielsweise das umgebende Wirtsmaterial, im folgenden Matrix genannt). Das heißt, der Akzeptor wird zum Radikalanion reduziert, also elektrisch dotiert.

Bei der erfindungsgemäßen Verwendung können als Endstufen einer erfolgreichen Dotierung mindestens folgende Komponenten generiert werden: ein sehr stabiles Kation, ein formell redox-inaktives Radikal und ein Radikalanion der umgebenden Matrix. Dabei vermag das redox-inaktive Radikal entweder als solches bestehen zu bleiben, in Lösung ggf. zu einem intermolekularen Folgeprodukt mit geschlossenen Elektronenschalen zu dimerisieren, oder es kann auch in besonderen Fällen (1,2-Diradikal) sich intramolekular absättigen zu einem Alken.

Prinzipiell kann die Dotierung über mehrere alternative Reaktionswege erfolgen, was von elektronischen und sterischen Gegebenheiten der eingesetzten Materialien abhängen kann:
1. Homolyse nach (bevorzugt) optischer Anregung in zwei Radikale (wie oben beschrieben). Spaltung in ein neutrales Radikal, welches bestrebt ist, sein Elektron an die Umgebung abzugeben, und in ein redoxinertes, stabiles Radikal.
2. Elektronenübertragung aus dem vollständigen Precursor durch (bevorzugt) optische Anregung und anschließender Zerfall des intermediär entstandenen Radikalkations in ein Kation und in ein redoxinertes, stabiles Radikal.
3. Heterolyse nach (bevorzugt) optischer Anregung in ein Kation und ein Anion; hierbei ist letzteres bestrebt, durch Elektronenabgabe in ein redoxinertes, stabiles Radikal überzugehen. Diese Spaltung kann bei hydridischen Varianten (also L = H) vorliegen.

Die Effektivität des Elektronentransfers, also die Dotandenstärke, richtet sich wesentlich nach drei Parametern:
Reduktionspotential des Kations (bevorzugt < -2.3V vs. Ferrocen/Ferrocen⁺, besonders bevorzugt < -2.6V, am meisten bevorzugt < -2.8V)

Tendenz der Abgangsgruppe, ein Radikal zu bilden. Beispielsweise ist dies bei Arylgruppen sehr gering ausgeprägt, so dass bei R = C₆H₅ eine Spaltung eher unwahrscheinlich ist. Umgekehrt ist Benzyl (also R = CH₂C₆H₅) ein effektiv stabilisiertes Radikal, so dass hier mit einer hohen Spaltungstendenz zu rechnen ist.

Stärke der C-L Bindung. Diese hängt von sterischen und elektronischen Gegebenheiten ab.

Das erfindungsgemäße Konzept bietet gegenüber dem Stand der Technik eine Reihe von Vorteilen, die im Folgenden erläutert werden: guter synthetischer Zugang; leichte Modifizierbarkeit; hohe Dotandenstärke, große Applikationsbreite.

Darüber hinaus weisen die Precursor eine hohe Stabilität gegenüber Sauerstoff und Feuchtigkeit auf (insbesondere im Vergleich zu Dotanden wie Dekamethylcobaltocen), so dass keine speziellen Schutzmaßnahmen (Inertgasatmosphäre) bei deren Verwendung getroffen werden müssen.

### Synthesen ausgewählter chemischer Verbindungen

Die Verbindungen sind über verschiedene Synthesewege zugänglich.

In der Literatur findet man die Umsetzung von geeigneten 1,2-Diaminobenzolen mit Aldehyden oder Ketonen, wobei durch Kondensation von Wasser die entsprechenden Imidazoline gebildet werden (G. W. H. Cheeseman, J. Chem. Soc. 1955,3308-3310).

Vorteilhafter wird jedoch erfindungsgemäß ein Imidazoliumion mit einem geeigneten Anion als starkem Nukleophil umgesetzt, wodurch der Dotand als Neutralverbindung resultiert. Dieser Weg weist gegenüber dem Stand der Technik die Vorzüge einer kürzeren Synthesesequenz und deutlich milderen Reaktionsbedingungen auf.

### Bevorzugte Synthesemethode:

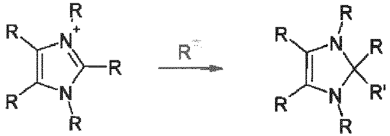

### Allgemeine Vorschrift: 2-Aminobenzimidazole

### 2- Aminobenzimidazolderivate

Die Synthese der 2-Aminobenzimidazolderivate aus geeigneten aromatischen Diaminen erfolgt wie in der Literatur beschrieben (W. Ried et al., J. Prakt. Chem. 1959, 8, 132-149).

### Imidazoliumionen

### Allgemeine Vorschrift: polycyclische Guanidiniumsalze

Überbrückung zu Guanidiniumderivaten erfolgt für alle Derivate gleichermaßen: Zu 5mmol des entsprechenden 2-Aminobenzimidazols werden in DMF 10mmol (2,0g) 1,3- Dibrompropan und 15mmol (1,9g) K₂CO₃ gegeben. Die Mischung wird unter Rühren für 5 h auf 60°C, dann 6h auf 90°C und letztlich weitere 6h auf 135°C erhitzt. Von Unlöslichem wird abfiltriert und das Filtrat eingeengt. Das Produkt fällt als Bromidsalz aus, welches noch im Vakuum getrocknet wird.

### Silyl Anionen

### Allgemeine Vorschrift: Alkalitriorganylsilyle als Reaktionsbausteine

Die Synthese erfolgt wie in der Literatur beschrieben (A. G. Brook et al., J. Am. Chem. Soc. 1954, 76, 2333-2338).

Weitere Metallorganica sind entweder käuflich (als Lithium- oder Magnesiumorganische Verbindung) oder werden in situ nach Standardmethoden dargestellt (z.B. Deprotonierung mit Buthyllithium in Diethylether)

### Dotandensynthesen

### A: Allgemeine Vorschrift für Dotanden mit L = H

Die Synthese erfolgt wie in der Literatur beschrieben (R. W. Alder et al., J. Chem. Soc. Chem. Commun. 1992,507-508)

### B: Allgemeine Vorschrift: 2,2-Dialkylimidazoline bzw. Orthocarbonsäureamide und Derivate

Das entsprechende Kation, beispielsweise ein Guanidiniumsalz, wird in THF suspendiert und unter Rühren bei RT mit einem metallorganischen Kupplungspartner versetzt. Der Umatz wird dadurch angezeigt, dass das unlösliche Salz aufgelöst und letztlich eine milchige Lösung erhalten wird. Diese Lösung wird mit Wasser versetzt und mit Diethylether extrahiert. Die Etherphase wird über Magnesiumsulfat getrocknet, filtriert und eingeengt. Man erhält das Produkt als farblosen Feststoff.

### Verbindung 11

### Synthese nach Vorschrift B

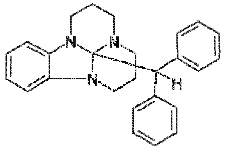

¹H-NMR (500MHz, THF-d₈): δ = 7.47, m, 4H; 7.05-7.25, m, 6H; 6.34, m, 2H; 6.23, m, 2H; 5.31, s, 1H; 3.24, m, 4H; 2.92, m, 2H; 2.54, m, 2H; 1.84, m, 2H; 1.21, m, 2H.

### Verbindung 12

### Synthese nach Vorschrift B

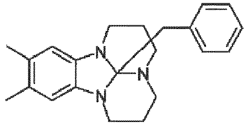

¹H-NMR (500MHz, THF-d₈): δ = 7.26, m, 2H; 7.10, m, 2H; 7.05, m, 1H; 6.06, s, 2H; 3.52, m, 2H; 3.46, s, 2H; 3.36, m, 2H; 2.98, m, 2H; 2.46, m, 2H; 2.04, s, 6H; 1.95, m, 2H; 1.32, m, 2H.

### Verbindung 13

### Synthese nach Vorschrift B

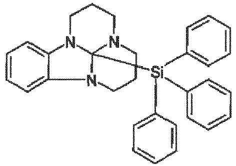

¹H-NMR (500MHz, THF-dg): δ = 7.78, m, 6H; 7.35, m, 3H; 7.28, m, 6H; 6.51, m, 2H; 6.34, m, 2H; 3.34, m, 2H; 3.04, m, 2H; 2.83, m, 4H; 1.83, m, 2H; 1.17, m, 2H.

### Verbindung 14

### Synthese nach Vorschrift A

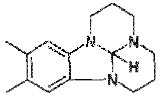

¹H-NMR (500MHz, CDCl₃): δ = 6.15, s, 2H; 4.47, s, 1H; 3.66, m, 2H; 3.15, m, 2H; 2.54, m, 2H; 2.25, m, 2H; 2.16, s, 6H; 1.80, m, 2H; 1.30, m, 2H.

### Verbindung 15

### Synthese nach Vorschrift A

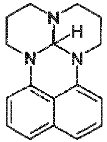

¹H-NMR (500MHz, CDCl₃): δ = 7.22, m, 2H; 7.09, m, 2H; 6.56, m, 2H; 4.58, s, 1H; 4.17, m, 2H; 3.05, m, 2H; 2.93, m, 2H; 2.38, m, 2H; 2.25, m, 2H; 1.39, m, 2H.

### Verbindung 16

### Synthese nach Vorschrift A

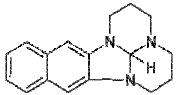

¹H-NMR (500MHz, CDCl₃): δ = 7.52, m, 2H; 7.15, m, 2H; 6.65, s, 2H; 4.63, s, 1H; 3.87, m, 2H; 3.29, m, 2H; 2.69, m, 2H; 2.36, m, 2H; 1.97, m, 2H; 1.47, m, 2H.

### Modifizierbarkeit

### Dotandenstärke:

Die effektive Dotandenstärke wird auf zwei Weisen abgestimmt: a) Reduktionspotential des Imidazoliumions oder Derivats und b) Radikalbildungstendenz der Abgangsgruppe L, bzw. Stärke der C-L-Bindung.

Im Test mit Benzophenon zeigte sich, dass L = CHPh₂ dieses Material dotiert (Blaufärbung durch Bildung des Radikalanions von Benzophenon), während keine sichtbare Reaktion mit L = CH₂Ph erfolgt, wohl aber wird in geeigneten Matrices eine Dotierwirkung erzielt (siehe unten). Mit L = Phenyl als besonders destabilisiertem Radikal wird praktisch keine nennenswerte Wirkung mehr erzielt (außer gegenüber starken Akzeptoren als direkter Dotand).

Bei gleichem Bau aber unterschiedlichen Reduktionspotentialen der Imidazoliumionen oder Derivaten wird mit **16** (CV: ca. -2.7V gegen Ferrocen) eine stärkere Wirkung als mit **15** (ca. 2.6V) erzielt (siehe Dotierexperimente).

### Flüchtigkeit:

Die Sublimationstendenz der erfindungsgemäß eingesetzten Verbindungen ist auf verschiede Arten leicht anzupassen.

Während **14** bereits im Ultrahochvakuum bei Raumtemperatur sublimiert, wurde in **16** der aktive Teil, in **12** der redoxinaktive Teil modifiziert (Einsatz der schweren Benzylgruppe, die durch geringen synthetischen Aufwand eingeführt wurde). Beide Verbindungen **16** und **12** sublimieren im Ultrahochvakuum deutlich höher (bei ca. 70°C) als **14.**

### Anorganische Abgangsgruppe L

Der redoxinaktive Teil L ist nicht auf organische Komponenten beschränkt: auch anorganische bzw. metallorganische L können mit geringem Aufwand eingeführt werden, wie mit Verbindung **13** demonstriert wird. Dieses Prinzip ist natürlich nicht auf Silizium beschränkt und kann auf andere Fragmente ausgeweitet werden.

So können prinzipiell auch Verbindungen mit L = OR und NR₂ (W. Kantlehner et al., J. Prakt. Chem. 2000, 342, 256-268) erfindungsgemäß eingesetzt werden.

### Prozessierbarkeit

Die relativ einfache Modifizierbarkeit durch Variation der Abgangsgruppe L ermöglicht die Anpassung an verschiedene Prozesse und Prozessparameter: Neben der Sublimationstemperatur wäre auch die Löslichkeit auf diese Weise einstellbar.

### Beschreibung der Dotierexperimente

Allgemein müssen die Reduktionspotentiale von n-Dotanden gewissen Ansprüchen genügen: Molekül und/oder neutrales Radikal mit einem HOMO Niveau kleiner als 3,3eV (bevorzugt kleiner als 2,8eV, mehr bevorzugt kleiner als 2,6eV) besteht. Das HOMO des Donatoren kann man aus cyclovoltammetrischen Messungen des Oxidationspotentials bestimmen. Alternativ kann das Reduktionspotential des Donatorkations in einem Salz des Donators bestimmt werden. Der Donator soll ein Oxidationspotential aufweisen, welches gegenüber Fc / Fc+ (Ferrocen/Ferrocenium Redoxpaar) kleiner oder gleich etwa -1,5 V, vorzugsweise kleiner oder gleich etwa -2,0 V, weiter bevorzugt kleiner oder gleich etwa -2,2 V ist.

Molare Masse des Donators zwischen 100 und 2000g/mol, bevorzugt zwischen 200 und 1000 g/mol.

Molare Dotierkonzentration zwischen 1:1000 (Akzeptormolekül:Matrixmolekül) und 1:2, bevorzugt zwischen 1:100 und 1:5, mehr bevorzugt zwischen 1:100 und 1:10. In Einzelfällen kommt auch ein Dotierverhältnis in Betracht, bei dem das Dotiermolekül mit einer höheren Konzentration als 1:2 angewendet wird, beispielsweise wenn eine besonders hohe Leitfähigkeit benötigt wird.

Der Donator kann sich während des Schichtherstellungsprozesses oder des darauf folgenden Schichtherstellungsprozesses aus einem Precursor (siehe DE 103 07 125.3) erst bilden. Das oben angegebene HOMO-Niveau des Donators bezieht sich dann auf die entstehende Spezies.

Der aktive Teil der erfindungsgemäß eingesetzten Materialien, also das Imidazoliumion oder Derivat, weist in cyclovoltammetrischen Messungen eine Reduktion zwischen -2.6V und - 2.9V (gegen Ferrocen als internen Standard) auf.

Daraus folgt, dass die erfindungsgemäß eingesetzten Dotanden (bzw. eigentlich die Precursoren) gegenüber älteren Verfahren, die auf einem vergleichbaren Konzept beruhen, über eine deutlich höhere Dotierleistung verfügen.

### Dotierexperiment 15 in ZnPc

**15** wird mit ZnPc im Verhältnis 1:10 koverdampft. Die Probe wird fünf Minuten in der evakuierten Anlage durch ein Quarzglasfenster mit einer Quecksilberdampflampe belichtet. Nach dem Ende der Belichtung lässt man das Substrat ca. 1h abklingen und misst dann die Leitfähigkeit.

Es wird eine Leitfähigkeit von 2x10⁻⁸ S/cm gemessen.

### Dotierexperiment 16 in ZnPc

**16** wird mit ZnPc im Verhältnis 1:10 koverdampft. Die Probe wird fünf Minuten in der evakuierten Anlage durch ein Quarzglasfenster mit einer Quecksilberdampflampe belichtet. Nach dem Ende der Belichtung lässt man das Substrat ca. 1h abklingen und misst dann die Leitfähigkeit.

Es wird eine Leitfähigkeit von 7x10⁻⁸ S/cm gemessen.

### Dotierexperiment 12 in ZnPc

**12** wird mit ZnPc im Verhältnis 1:10 koverdampft. Die Probe wird fünf Minuten in der evakuierten Anlage durch ein Quarzglasfenster mit einer Quecksilberdampflampe belichtet. Nach dem Ende der Belichtung lässt man das Substrat ca. 1h abklingen und misst dann die Leitfähigkeit.

Es wird eine Leitfähigkeit von 9x10⁻⁸ S/cm gemessen.

Besonders vorteilhaft kann die Dotandenstärke in Lösung mit Benzophenon deutlich gemacht werden: Diese Verbindung wird erst bei ca. -2.3V (gegen Ferrocen als internen Standard) reduziert. Dieser Wert entspricht dem Reduktionspotential der meisten Matrixmaterialien, sodass hier eine aussagekräftige Modellverbindung vorliegt.

Die Reduktion von Benzophenon zum Radikalanion (also eine geglückte "Dotierung") wird durch eine intensiv blaue Färbung indiziert.

### Demonstration der Dotierstärke: Lösung von 11 mit Benzophenon in THF

In einer argongefluteten Glovebox wird eine kleine Probe von **11** mit einem zehnfachen Gewichtsüberschuss Benzophenon und ca. 3ml absolutiertem THF in ein luftdicht abschließbares NMR-Röhrchen gegeben, ausgeschleust und ca. 10 min unter eine handelsübliche Labor-UV-Lampe (366nm) gehalten. Die persistente Blaufärbung der Lösung zeigt den erfolgreichen Elektronentransfer vom n-Dotanden auf den schwachen Akzeptor Benzophenon an. Wird das Röhrchen geöffnet und die Lösung ausgegossen, wird sie augenblicklich durch den Luftkontakt entfärbt.

### Demonstration der Dotierstärke: Lösung von 13 mit Benzophenon in THF

In einer argongefluteten Glovebox wird eine kleine Probe von **13** mit einem zehnfachen Gewichtsüberschuss Benzophenon und ca. 3ml absolutiertem THF in ein luftdicht abschließbares NMR-Röhrchen gegeben, ausgeschleust und ca. 10 min unter eine handelsübliche Labor-UV-Lampe (366nm) gehalten. Die persistente Blaufärbung der Lösung zeigt den erfolgreichen Elektronentransfer vom n-Dotanden auf den schwachen Akzeptor Benzophenon an. Wird das Röhrchen geöffnet und die Lösung ausgegossen, wird sie augenblicklich durch den Luftkontakt entfärbt.

### Demonstration der Dotierstärke: Verreibung von 14 mit Benzophenon

In einer argongefluteten Glovebox wird eine kleine Probe von **14** mit einem zehnfachen Gewichtsüberschuss Benzophenon in einem kleinen Achatmörser fein verrieben und in ein luftdicht abschließbares NMR-Röhrchen gegeben, ausgeschleust und ca. 10 min unter eine handelsübliche Labor-UV-Lampe (366nm) gehalten. Die Blaugrünfärbung der festen Mischung zeigt den erfolgreichen Elektronentransfer vom n-Dotanden auf den schwachen Akzeptor Benzophenon an. Wird das Röhrchen geöffnet und geleert, wird das Pulver augenblicklich durch den Luftkontakt entfärbt.

### Prozessierung

### Herstellung dotierter Schichten

Die niedrig-molekularen Schichten werden typischerweise durch ein Vakuumverfahren aufgedampft, wie z.B. VTE (vacuum thermal evaporation) oder OVPD (organic vapour phase deposition). Des Weiteren können Vakuum-Spray Verfahren zum Einsatz kommen.

Eine weitere Abscheidungsart umfasst den thermisch oder optisch induzierten Übertrag des Materials von einem Trägersubstrat auf das eigentliche Substrat (vgl. z.B. LITI: laser induced thermal imaging).

Dotierte Schichten werden im Vakuum typischerweise mittels Mischverdampfung aus zwei unabhängig geregelten Quellen (für Matrixmaterial und Dotand) hergestellt. Sie können alternativ dazu auch durch Interdiffusion aus einer Dotandenschicht in die darunter liegende Matrixmaterialschicht entstehen, wobei die beiden Materialien also nacheinander im Vakuum aufgedampft werden. Die Interdiffusion kann thermisch gesteuert sein. Unter Umständen muss der Dotand noch während des Herstellungsprozesses oder in der Schicht durch geeignete physikalische und/oder chemische Maßnahmen (z.B. Lichteinwirkung, Einwirkung von magnetischen und/oder elektrischen Feldern) aktiviert werden.

Alternative Herstellungsmethoden für dotierte Schichten sind weiterhin:
- Mischverdampfung eines Gemisches von Matrix und Dotand aus ein und derselben Quelle.
- Dotierung einer Matrixschicht durch eine Lösung von Dotanden mit anschließendem Verdampfen des Lösungsmittels, insbesondere durch thermische Behandlung.
- Oberflächendotierung einer Matrixmaterialschicht durch eine oberflächlich aufgebrachte Schicht von Dotanden.
- Herstellung einer Lösung von Matrixmolekülen und Dotanden und anschließende Herstellung einer Schicht aus dieser Lösung mittels konventioneller Methoden wie beispielsweise Verdampfen des Lösungsmittels oder Aufschleudern.

### Geeignete Matrixmaterialien

Als Matrixmaterialien für Elektronentransportschichten könnten beispielweise Fullerene, wie beispielsweise C60, Oxadiazolderivate, wie beispielsweise 2-(4-Biphenyl)-5-(4-tert-butylphenyl)-1,3,4-oxadiazol, Chinoxalinbasierte Verbindungen wie beispielsweise Bis(phenylchinoxaline), oder Oligothiophene, Perylenderivate, wie z.B. Perylentetracarbonsäuredianhydrid, Naphthalenderivate, wie z.B. Naphthalintetracarbonsäuredianhydrid, oder andere Elektronentransportmaterialien eingesetzt werden, wie sie z.B. in A.P. Kulkarni et al., Chem. Mater. 2004, 16,4556 beschrieben sind.

Als Matrixmaterialien für Elektronentransportschichten können weiterhin Quinolinatokomplexe, beispielsweise des Aluminiums oder anderer Hauptgruppenmetalle, wobei der Quinolinatoligand auch substituiert sein kann, eingesetzt werden. Insbesondere kann das Matrixmaterial Tris(8-hydroxy-quinolinato)-aluminium sein. Auch andere Aluminiumkomplexe mit O- und/oder N- Donoratomen können gegebenenfalls eingesetzt werden. Die Quinolinatokomplexe können beispielsweise einen, zwei oder drei Quinolinatoliganden enthalten, wobei die anderen Liganden vorzugsweise mit O- und/oder N-Donoratomen an das Zentralatom komplexieren, wie beispielsweise untenstehender Al-Komplex.

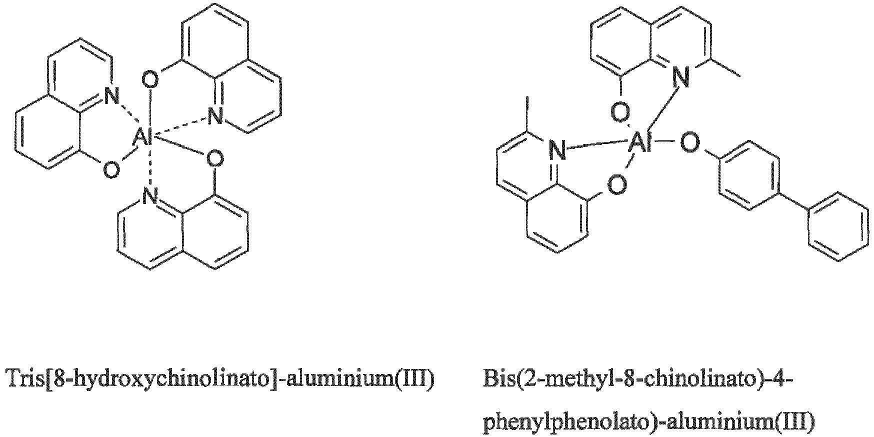

Als Matrixmaterial können auch Phenanthroline eingesetzt werden, die substituiert oder unsubstituiert sein können, insbesondere Aryl-substituiert, beispielsweise Phenyl- oder Naphthyl-substituiert, oder auch beispielsweise Bathocuproin (BCP). Insbesondere kann Bathophenanthrolin (Bphen) als Matrixmaterial eingesetzt werden.

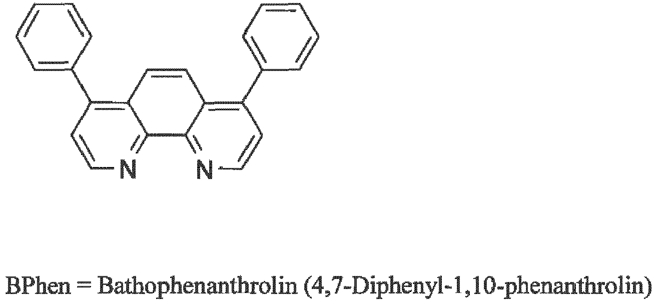

Desweiteren können als Matrixmaterialien auch Heteroaromaten wie insbesondere Triazolderivate eingesetzt werden, gegebenenfalls auch Pyrrole, Imidazole, Triazole, Pyridine, Pyrimidine, Pyridazine Chinoxaline, Pyrazino-chinoxaline und dergleichen. Die Heteroaromaten sind vorzugsweise substituiert, insbesondere Aryl-substituiert, beispielsweise Phenyl- oder Naphthyl-substituiert. Insbesondere kann untenstehendes Triazol als Matrixmaterial eingesetzt werden.

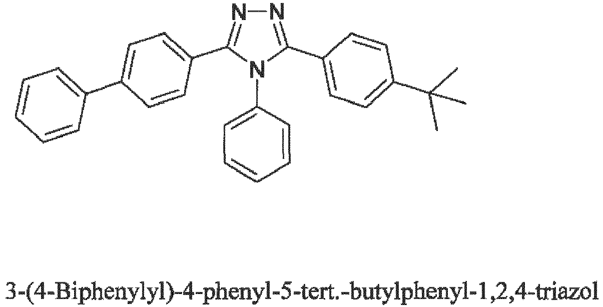

Es versteht sich, dass die genannten Matrixmaterialien auch untereinander oder mit anderen Materialien gemischt im Rahmen der Erfindung einsetzbar sind. Es versteht sich, dass auch geeignete andere organische Matrixmaterialien verwendet werden können, die halbleitende Eigenschaften aufweisen.

### Verwendungszweck

Unter Verwendung der beschriebenen Verbindungen zur Herstellung organischer halbleitender Materialien, die insbesondere in Form von Schichten oder elektrischen Leitungspfaden angeordnet sein können, können eine Vielzahl elektronischer Bauelemente oder diese enthaltende Einrichtungen mit einer dotierten organischen Halbleiterschicht hergestellt werden. Im Sinne der Erfindung werden von dem Begriff "elektronische Bauelemente" auch optoelektronische Bauelemente mit umfasst. Durch die beschriebenen Verbindungen können die elektronischen Eigenschaften eines elektronisch funktionell wirksamen Bereichs des Bauelementes, wie dessen elektrische Leitfähigkeit, lichtemittierende Eigenschaften oder dergleichen, vorteilhaft verändert werden. So kann die Leitfähigkeit der dotierten Schichten verbessert und/oder die Verbesserung der Ladungsträgerinjektion von Kontakten in die dotierte Schicht erreicht werden.

Die Erfindung umfasst insbesondere organische lichtemitierende Dioden (OLED), organische Solarzellen, organische Dioden, insbesondere solche mit hohem Gleichrichtungsverhältnis wie 10³-10⁷,vorzugsweise 10⁴-10⁷ oder 10⁵-10⁷, und organische Feldeffekttransistoren, die erfindungsgemäß hergestellt werden können.

In dem elektronischen Bauelement kann eine n-dotierte Schicht auf Basis eines organischen Matrixmaterials beispielsweise in folgenden Schichtstrukturen vorliegen, wobei vorzugsweise die Basismaterialien oder Matrixmaterialien der einzelnen Schichten jeweils organisch sind:
- M-i-n: Metall - Isolator - n-dotierter Halbleiter, wobei die Schicht M den Metallgrundkontakt bildet und beispielsweise ITO, Au, Ag, Al usw. sein kann. Der Deckkontakt bildet mit der n-dotierten Schicht einen ohmschen Kontakt und kann beispielsweise aus Al bestehen. Die Schicht "i" steht für eine undotierte Schicht.
- n-i-M: es gelten die Ausführungen zur M-i-n Struktur, im Unterschied hierzu ist jedoch der ohmsche Kontakt auf dem Substrat vorgesehen.
- p-i-n: p-dotierter Halbleiter - Isolator - n-dotierter Halbleiter,
- n-i-p: n-dotierter Halbleiter - Isolator - p-dotierter Halbleiter.

"i" ist wiederum eine undotierte Schicht, "p" ist eine p-dotierte Schicht. Die Kontaktmaterialien sind hier löcherinjizierend, wobei p-seitig beispielsweise eine Schicht oder ein Kontakt aus ITO oder Gold vorgesehen sein kann, oder elektroneninjizierend, wobei n-seitig eine Schicht oder ein Kontakt aus ITO, Aluminium oder Silber vorgesehen sein kann. In obigen Strukturen kann im Bedarfsfall auch die i-Schicht ausgelassen werden, wodurch Schichtenabfolgen mit p-n oder n-p-Übergängen erhalten werden können.

Die Verwendung der beschriebenen Verbindungen ist jedoch auf die oben genannten Ausführungsbeispiele nicht beschränkt, insbesondere können die Schichtstrukturen durch Einführung zusätzlicher geeigneter Schichten ergänzt bzw. modifiziert werden.

Die erfindungsgemäßen Verbindungen und deren Derivate können erfindungsgemäß in den elektronischen Bauelementen aber auch in Schichten, Leitfahigkeitspfaden, Punktkontakten oder dergleichen eingesetzt werden, wenn diese gegenüber einer anderen Komponente überwiegen, beispielsweise als Injektionsschicht in reiner oder im wesentlichen reiner Form.

In einer anderen Konfiguration kann der erfindungsgemäße n-Dotand auch zur Verbesserung der Injektion aus der Kathode eingesetzt werden. Dazu ist vorgesehen, eine Schicht, im Wesentlichen bestehend aus dem Dotanden, zwischen die Kathode und eine elektronentransportierende Schicht zu bilden. Eine Schicht, im wesentlichen bestehend aus dem Dotanden, kann eine Schichtdicke größer als 0,5 nm aufweisen, bevorzugt zwischen 0,5 nm und 20nm, mehr bevorzugt zwischen 1nm und 10nm, noch mehr bevorzugt zwischen 1nm und 3nm. Eine Schicht, im Wesentlichen bestehend aus dem Dotanden, kann als reine Schicht des Dotanden ausgeführt sein.

Bei der Prozessierung aus Lösung können fertige Formulierungen aus Matrix (ggf. auch einem halbleitenden Polymer) und Dotand in Lösung bereitgestellt werden.

Denkbar ist auch eine Formulierung von Monomer und Dotand, wobei nach Aktivierung des Dotanden die freiwerdenden Radikale eine Polymerisation der Monomere starten.

Außerdem ist es leicht möglich, weitere Funktionalitäten zu kombinieren, z.B. Kopplung des Dotanden an Wirtsmaterialien.

Die in der vorstehenden Beschreibung und in den Ansprüchen offenbarten Merkmale der Erfindung können sowohl einzeln als auch in jeder beliebigen Kombination für die Verwirklichung der Erfindung in ihren verschiedenen Ausführungsformen wesentlich sein.

## Patentansprüche

1. Verwendung eines Precursors eines n-Dotanden zur Dotierung eines organischen halbleitenden Materials, als Blockerschicht, als Ladungsinjektionsschicht, als Elektrodenmaterial, als Speichermaterial oder als Halbleitermaterial selbst in elektronischen oder optoelektronischen Bauelementen, wobei der Precursor ausgewählt wird aus den folgenden Formeln : 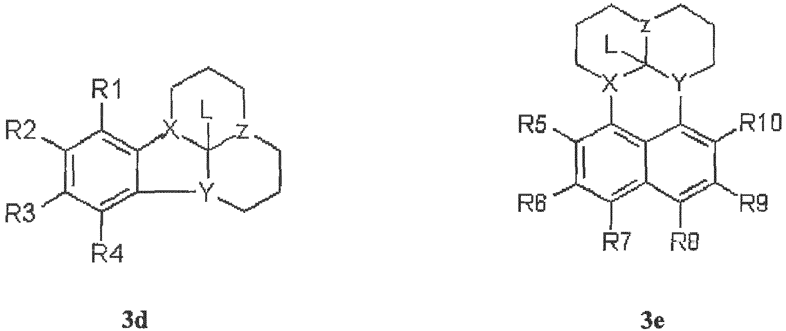 wobei
L H, eine organische, anorganische oder metallorganische Abgangsgruppe darstellt;
X, Y und Z unabhängig N oder P sind;
R₁-R₁₀ unabhängig ausgewählt werden aus H, C₁-C₁₀-Alkyl und C₆-C₁₂-Aryl.

2. Verwendung nach Anspruch 1, **dadurch gekennzeichnet, dass** L ausgewählt wird aus H, CR₃, OR, NR₂ und SiR₃, wobei jedes R unabhängig ausgewählt ist aus H, Alkyl und Aryl; wobei L bevorzugt ausgewählt wird aus H, sec-Alkyl, tert-Alkyl, CH₂Ar, CHAr₂, SiAr₃, SiAr₂-alkyl und SiAr₁-alkyl₂ mit Ar=Aryl, wobei Ar = Phenyl, Thienyl, Furanyl; sec-Alkyl = Cyclohexyl, Cyclopentyl, 2-Butyl, 2-Propyl; tert.-Alkyl = tert-Butyl, Adamantyl bevorzugt sind.

3. Verwendung nach Anspruch 1 oder 2, **dadurch gekennzeichnet, dass** der Precursor durch Aktivierung mit elektromagnetischer Strahlung in einen n-Dotanden umgesetzt wird.

4. Verwendung nach Anspruch 3, **dadurch gekennzeichnet, dass** die elektromagnetische Strahlung sichtbares Licht, UV-Licht oder IR-Licht ist.

5. Verbindung nach einer der Formeln: 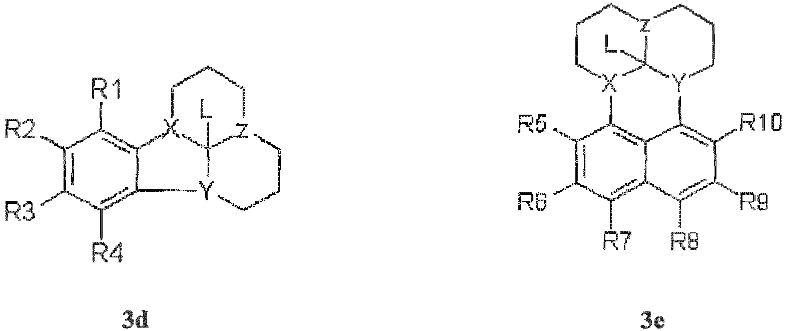 mit L, X, Y und Z und R₁-R₁₀ wie in Anspruch 1, wobei Verbindung 3e mit L=H und R₅-R₁₀=H ausgeschlossen ist.

6. Organisches halbleitendes Material enthaltend zumindestens eine organische Matrixverbindung und einen Dotanden, **dadurch gekennzeichnet, dass** als Dotand zumindest eine Verbindung gemäß den Ansprüchen 1 bis 4 verwendet wird.

7. Organisches halbleitendes Material nach Anspruch 6, **dadurch gekennzeichnet, dass** das molare Dotierungsverhältnis von Dotand zu Matrixmolekül bzw. das Dotierungsverhältnis von Dotand zu monomeren Einheiten eines polymeren Matrixmoleküls zwischen 20:1 und 1:100.000, bevorzugt 10:1 und 1:1.000, besonders bevorzugt 1:1 und 1:100, beträgt.

8. Elektronisches oder optoelektronisches Bauelement mit einem elektronisch funktionell wirksamen Bereich, **dadurch gekennzeichnet, dass** für den elektronisch wirksamen Bereich zumindest eine Verbindung der Ansprüche 1 bis 4 verwendet wird.

9. Elektronisches oder optoelektronisches Bauelement nach Anspruch 8, **dadurch gekennzeichnet, dass** der elektronisch wirksame Bereich ein organisches halbleitendes Matrixmaterial aufweist, welches mit zumindest einem Dotanden zur Veränderung der elektronischen Eigenschaften des halbleitenden Matrixmaterials unter Verwendung zumindest einer Verbindung der Ansprüche 1 bis 4 dotiert wird.

10. Elektronisches oder optoelektronisches Bauelement nach Anspruch 8 oder 9 in Form einer organischen licht-emittierenden Diode, einer photovoltaischen Zelle, einer organischen Solarzelle, einer organischen Diode, eines organischen Feldeffekttransistors oder eines photoinitiierten bzw. magnetischen Speichers.

## Claims

1. Use of a precursor of an n-dopant for doping an organic semiconducting material, as a blocking layer, as a charge injection layer, as an electrode material, as a storage material or as a semiconducting material itself, in electronic or optoelectronic components, wherein the precursor is selected from the following formulae: 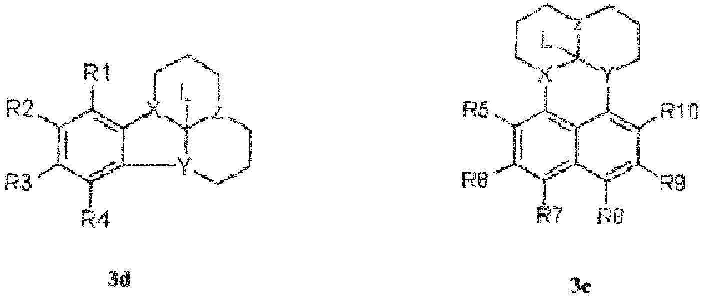 wherein
L represents H or an organic, inorganic or organometallic leaving group;
X, Y and Z, independently of one another, are N or P;
R₁-R₁₀ are independently selected from H, C₁-C₁₀ alkyl and C₆-C₁₂ aryl.

2. Use as claimed in claim 1, **characterized in that** L is selected from H, CR₃, OR, NR₂ and SiR₃, wherein each R is independently selected from H, alkyl and aryl; wherein L is preferably selected from H, sec-alkyl, tert-alkyl, CH₂Ar, CHAr₂, SiAr₃, SiAr₂-alkyl and SiAr₁-alkyl₂ in which Ar = aryl, wherein Ar = phenyl, thienyl, furanyl; sec-alkyl = cyclohexyl, cyclopentyl, 2-butyl, 2-propyl; tert-alkyl = tert-butyl, adamantyl are preferred.

3. Use as claimed in claim 1 or claim 2, **characterized in that** the precursor is converted into an n-dopant by activation with electromagnetic radiation.

4. Use as claimed in claim 3, **characterized in that** the electromagnetic radiation is visible light, UV light or IR light.

5. A compound with one of the formulae: 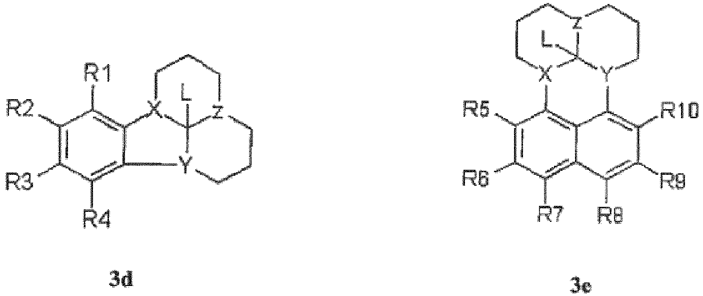 in which L, X, Y and Z and R₁-R₁₀ are as claimed in claim 1, wherein compound 3e in which L=H and R₅-R₁₀=H is excluded.

6. An organic semiconducting material containing at least one organic matrix compound and a dopant, **characterized in that** at least one compound as claimed in claims 1 to 4 is used as the dopant.

7. The organic semiconducting material as claimed in claim 6, **characterized in that** the molar doping ratio of dopant to matrix molecule or the doping ratio of dopant to monomeric units of a polymeric matrix molecule is between 20:1 and 1:100000, preferably 10:1 and 1:1000, particularly preferably 1:1 and 1:100.

8. An electronic or optoelectronic component with an electronically functionally active region, **characterized in that** at least one compound as claimed in claims 1 to 4 is used as the electronically active region.

9. The electronic or optoelectronic component as claimed in claim 8, **characterized in that** the electronically active region comprises an organic semiconducting matrix material which is doped with at least one dopant in order to modify the electronic properties of the semiconducting matrix material using at least one compound as claimed in claims 1 to 4.

10. The electronic or optoelectronic component as claimed in claim 8 or claim 9, in the form of an organic light-emitting diode, a photovoltaic cell, an organic solar cell, an organic diode, an organic field effect transistor or a photo-initiated or magnetic storage means.

## Revendications

1. Utilisation d'un précurseur d'un dopant de type n, destiné à doper un matériau semi-conducteur organique, en tant que couche de blocage, en tant que couche d'injection de charges, en tant que matériau d'électrode, en tant que matériau de stockage ou en tant que matériau semi-conducteur proprement dit, dans des composants électroniques ou optoélectroniques, ledit précurseur étant choisi parmi les formules suivantes: 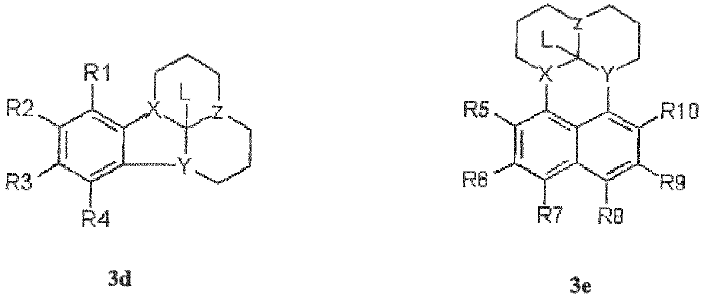 dans lesquelles
L représente H, un groupe partant organique, inorganique ou organométallique ;
X, Y et Z représentent, de façon indépendante, N ou P ;
R₁-R₁₀ sont choisis, de façon indépendante, parmi H, alkyle en C₁-C₁₀ et aryle en C₆-C₁₂.

2. Utilisation selon la revendication 1, **caractérisée en ce que** L est choisi parmi H, CR₃, OR, NR₂ et SiR₃, chaque R étant choisi, de façon indépendante, parmi H, alkyle et aryle; L étant préférentiellement choisi parmi H, sec.-alkyle, tert.-alkyle, CH₂Ar, CHAr₂, SiAr₃, SiAr₂-alkyle et SiAr₁-alkyle₂, avec Ar = aryle, Ar correspondant préférentiellement à phényle, thiényle, furanyle; sec.-Alkyle à cyclohexyle, cyclopentyle, 2-butyle, 2-propyle; tert.-alkyle à butyle, adamantyle.

3. Utilisation selon les revendications 1 ou 2, **caractérisée en ce que** l'on fait réagir ledit précurseur, par activation au moyen d'un rayonnement électromagnétique, pour obtenir un dopant de type n.

4. Utilisation selon la revendication 3, **caractérisée en ce que** ledit rayonnement électromagnétique est de la lumière visible, du rayonnement UV ou du rayonnement IR.

5. Composé selon l'une des formules : 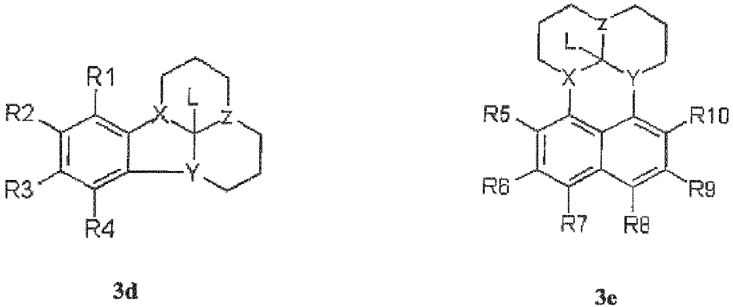 dans lesquelles L, X, Y et Z correspondent aux indications dans la revendication 1, les options L = H et R₅-R₁₀ = H étant exclues pour le composé 3e.

6. Matériau semi-conducteur organique contenant au moins un composé de matrice organique et un dopant, **caractérisé en ce que** l'on utilise, en tant que dopant, au moins un composé selon les revendications 1 à 4.

7. Matériau semi-conducteur organique selon la revendication 6, **caractérisé en ce que** le rapport molaire de dopage établi entre le dopant et la molécule de matrice ou, le cas échéant, le rapport de dopage établi entre le dopant et des unités monomères d'une molécule de matrice polymère, se situe entre 20 : 1 et 1 : 100 000, de préférence entre 10 : 1 et 1 : 1000, avec une préférence particulière entre 1 : 1 et 1 : 100.

8. Composant électronique ou optoélectronique comportant une partie pourvue d'une fonctionnalité électronique, **caractérisé en ce que** l'on utilise au moins un composé des revendications 1 ou 4 pour la partie ayant une activité électronique.

9. Composant électronique ou optoélectronique selon la revendication 8, **caractérisé en ce que** la partie ayant une activité électronique comporte un matériau de matrice semi-conducteur organique qui est dopé, en utilisant au moins un composé des revendications 1 à 4, avec au moins un dopant afin de modifier les propriétés électroniques dudit matériau de matrice semi-conducteur.

10. Composant électronique ou optoélectronique selon les revendications 8 ou 9 sous la forme d'une diode électroluminescente organique, d'une cellule photovoltaïque, d'une cellule solaire organique, d'une diode organique, d'un transistor à effet de champ organique ou d'une mémoire à photo-initiation et/ou de type magnétique.
